# FASCICULE DE BREVET EUROPEEN

(11) **EP 3 390 344 B1**
(45) Date de publication et mention de la délivrance du brevet: **02.12.2020**
(21) Numéro de dépôt: 16808712.0
(22) Date de dépôt: 12.12.2016
(51) Int. Cl.: C07C 209/16, C07C 213/04, C07C 213/08, C07C 231/02, C07C 249/02, C07C 211/12, C07C 251/08, C07C 233/05, C07C 233/18, C07C 209/50

(54) **PROCEDE AMELIORE DE SYNTHESE DE 1,6-HEXANEDIAMINES TERTIAIRES PARTIELLEMENT N-HYDROXYETHYLEES**
VERBESSERTES VERFAHREN ZUR SYNTHESE VON TEILWEISE N-HYDROXYETHYLIERTEN TERTIÄREN 1,6-HEXANEDIAMINEN
IMPROVED METHOD FOR SYNTHESISING PARTIALLY N-HYDROXYETHYLATED TERTIARY 1,6-HEXANEDIAMINES

(30) Priorité: 17.12.2015 FR 1562671
(43) Date de publication de la demande: 24.10.2018
(73) Titulaire: IFP Energies nouvelles, 92500 Rueil-Malmaison (FR)
(72) Inventeur: DELFORT, Bruno, 75005 Paris (FR); GRANDJEAN, Julien, 69008 Lyon (FR)
(74) Mandataire: IFP Energies nouvelles
(86) Numéro de dépôt international: PCT/EP2016/080669
(87) Numéro de publication internationale: WO 2017/102659

(56) Documents cités:
- US-A1- 2005 164 903
- M. Ishidate: "Studies on Carcinostatic Substances. XVIII. Anticancer Action of N, N'-Bis (2-chloroethyl)-N, N'-dimethylpolymethylenediamines", Chemical and Pharmaceutical Bulletin, 1 janvier 1958 (1958-01-01), pages 164-169, XP055291401, DOI: 10.1248/cpb.6.164 Extrait de l'Internet: URL:https://www.jstage.jst.go.jp/article/c pb1958/6/2/6_2_164/_pdf [extrait le 2016-07-26] cité dans la demande
- CLARKE H T ET AL: "The Action of Formaldehyde on Amines and Amino Acids", JOURNAL OF THE AMERICAN CHEMICAL SOCIETY, AMERICAN CHEMICAL SOCIETY, US, vol. 55, no. 11, 1 janvier 1933 (1933-01-01), pages 4571-4587, XP002402949, ISSN: 0002-7863, DOI: 10.1021/JA01338A041
- NIE J ET AL: "Synthesis and photopolymerization of N,N'-dimethyl,-N,N'-di(methacryloxy ethyl)-1,6-hexanediamine as a polymerizable amine coinitiator for dental restorations", BIOMATERIALS, ELSEVIER SCIENCE PUBLISHERS BV., BARKING, GB, vol. 23, no. 4, 15 février 2002 (2002-02-15), pages 1221-1226, XP004348141, ISSN: 0142-9612, DOI: 10.1016/S0142-9612(01)00241-1 cité dans la demande

## Description

### Domaine de l'invention

La présente invention concerne la synthèse de 1,6-hexanediamines tertiaires partiellement N-hydroxyéthylées.

### Contexte général

Les 1,6-hexanediamines tertiaires partiellement N-hydroxyéthylées peuvent présenter de l'intérêt pour différentes applications.

Les 1,6-hexanediamines tertiaires partiellement N-hydroxyéthylées peuvent être avantageusement utilisées par exemple dans la désacidification de gaz acides que ce soit le gaz naturel ou des gaz de fumées de combustion. On entend par désacidification de gaz acides la réduction de la teneur de ces gaz en composés acides tels que l'hydrogène sulfuré (H₂S), le dioxyde de carbone (CO₂), l'oxysulfure de carbone (COS), le disulfure de carbone (CS₂).

Les 1,6-hexanediamines tertiaires partiellement N-hydroxyéthylées peuvent entrer dans des formulations conduisant à des polymères, notamment les polyuréthanes.

Les 1,6-hexanediamines tertiaires partiellement N-hydroxyéthylées peuvent être des composés précurseurs de molécules trouvant des applications dans d'autres domaines de la chimie.

Parmi les 1,6-hexanediamines tertiaires partiellement N-hydroxyéthylées, on connait la synthèse de la N,N'-diméthyl-N,N'-di(2-hydroxyéthyl)-1,6-hexanediamine de formule **(I₂)** ci-dessous, faisant appel à l'utilisation de réactifs précurseurs halogénés.

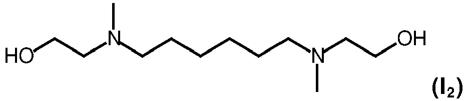

Le document US 2005/0164903 décrit par exemple la synthèse de la N,N'-diméthyl-N,N'-di(2-hydroxyéthyl)-1,6-hexanediamine par une réaction de condensation entre une mole de 1,6-dichlorohexane et deux moles de 2-(méthylamino)éthanol. Cette réaction de condensation conduit de manière inévitable à deux moles d'acide chlorhydrique. La purification de l'amine impose de convertir l'acide chlorhydrique en sel par réaction avec une base, par exemple de le convertir en chlorure de sodium après réaction avec de la soude ou avec du carbonate de sodium.

Ishidate et al, 1958 (« Studies on carcinostatic substances. XVIII. Anticancer action of N,N'-bis(2-chloroethyl)-N,N'-dimethylpolymethylenediamines », Chemical & Pharmaceutical Bulletin (1958), vol.6, pp. 164-169) décrivent la synthèse de la N,N'-diméthyl-N,N'-di(2-hydroxyéthyl)-1,6-hexanediamine par une réaction de condensation entre une mole de 1,6-dibromohexane et deux moles de 2-(méthylamino)éthanol. Cette réaction de condensation produit de manière inévitable deux moles d'acide bromhydrique. La purification de l'amine impose alors de convertir l'acide bromhydrique en sel par réaction avec une base, par exemple de le convertir en bromure de sodium après réaction avec de la soude ou avec du carbonate de sodium.

L'article de Nie et Bowman, 2002 (« Synthesis and photopolymerization of N,N'-dimethyl,-N,N'-di(methacryloxyethyl)-1,6-hexanediamine as a polymerizable amine coinitiator for dental restorations » Biomaterials 23, 2002, pp 1221-1226) décrit la synthèse de la N,N'-diméthyl-N,N'-di(2-hydroxyéthyl)-1,6-hexanediamine par une réaction de condensation entre la N,N'-diméthyl-1,6-hexanediamine et le 2-bromoéthanol en présence de soude. Cette voie de synthèse génère deux moles de bromure de sodium par mole de N,N'-diméthyl-N,N'-di(2-hydroxyéthyl)-1,6-hexanediamine.

Ishidate et al, 1958 (« Studies on carcinostatic substances. XVIII. Anticancer action of N,N'-bis(2-chloroethyl)-N,N'-dimethylpolymethylenediamines », Chemical & Pharmaceutical Bulletin (1958), vol.6, pp. 164-169) décrivent également la synthèse de la N,N'-diméthyl-N,N'-di(2-hydroxyéthyl)-1,6-hexanediamine en 4 étapes à partir de la 1,6-hexanediamine, dont une première étape qui est la réaction entre une mole de 1,6-hexanediamine et deux moles de chlorure de tosyle ou chlorure de p-toluenesulfonyle pour conduire à une mole de N,N'-diméthyl-N,N'-ditosyl-1,6-hexanediamine et deux moles d'acide chlorhydrique. La purification du produit de la réaction impose de convertir l'acide chlorhydrique en sel par réaction avec une base, par exemple de le convertir en chlorure de sodium, après réaction avec de la soude ou avec du carbonate de sodium.

Ishidate et al, 1958 (« Studies on carcinostatic substances. XVIII. Anticancer action of N,N'-bis(2-chloroethyl)-N,N'-dimethylpolymethylenediamines », Chemical & Pharmaceutical Bulletin (1958), vol.6, pp. 164-169) décrivent aussi la synthèse de la N,N'-diméthyl-N,N'-di(2-hydroxyéthyl)-1,6-hexanediamine en 3 étapes à partir de la 1,6-dibromohexane, dont une première étape qui est la réaction entre une mole de 1,6-dibromohexane et deux moles de tosylate de méthylamine ou N-méthyl-p-toluènesulfonamide pour conduire à une mole de N,N'-di(p-tosyl)hexanediamine et à deux moles d'acide bromohydrique. La purification de l'amine impose de convertir l'acide bromhydrique en sel par réaction avec une base, par exemple de le convertir en bromure de sodium, après réaction avec de la soude ou avec du carbonate de sodium.

Dans les modes de synthèse de l'art antérieur cité, les réactions produisent des sels parallèlement aux produits souhaités. Les sels formés doivent être séparés du milieu par exemple par filtration ou par centrifugation ou par lavage. Ces voies de synthèse qui imposent la gestion et l'élimination d'une quantité importante de sels ne remplissent pas les conditions requises de nos jours en vue d'une chimie verte et durable. L'extension de ces voies de synthèse à une échelle industrielle n'est donc pas souhaitable.

### Objectifs et résumé de l'invention

Les inventeurs ont mis en évidence qu'il était possible de préparer certaines 1,6-hexanediamines tertiaires partiellement N-hydroxyéthylées, répondant à la formule générale (I) telle que définie plus bas, selon une procédure ne mettant en œuvre aucun composé halogéné, tel par exemple le 1,6-dichlorohexane ou le 1,6-bromohexane ou un autre 1,6-dihalohexane, ni aucun autre composé halogéné tel par exemple le 2-bromoéthanol ou un autre 2-haloéthanol, ni aucun réactif appartenant à la famille des halogénures de tosyle tel par exemple le chlorure de tosyle. Les inventeurs ont mis en évidence qu'il était possible de préparer ces 1,6-hexanediamines tertiaires partiellement N-hydroxyéthylées selon une procédure ne générant aucun sel, ce qui est favorable pour envisager un développement industriel.

Le procédé de synthèse selon l'invention fait systématiquement appel à des produits de départ précurseurs exempts d'atomes d'halogènes et repose sur des réactions ne faisant intervenir aucun réactif organique halogéné.

En particulier, le procédé de synthèse selon l'invention fait appel à un précurseur exempt d'atome d'halogène et qui présent un squelette carboné qui est un enchaînement linéaire de 6 atomes de carbone, dans lequel les atomes de carbone situés en position 1 et 6, c'est-à-dire en alpha et en oméga, ne sont pas liés à un atome d'halogène tel par exemple un atome de chlore ou de brome.

Ce précurseur présente un enchaînement linéaire de 6 atomes de carbones dont les 4 atomes de carbone centraux sont liés chacun à 2 atomes d'hydrogène, et les 2 atomes de carbone situés en position 1 et 6, c'est-à-dire en alpha et en oméga, sont liés chacun, par une liaison simple à un atome d'azote : le précurseur est la 1,6-hexanediamine.

En plus de ce précurseur, les autres produits utilisés pour conduire à la N,N'-diméthyl-N,N'-di(2-hydroxyéthyl)-1,6-hexanediamine de formule (I₂) ne sont en aucun cas des composés organiques halogénés.

Le composé selon la formule (I₂) peut être obtenu à partir de ce précurseur en 2 étapes de réactions qui ne produiront aucun acide hydrohalogéné.

Les réactions mises en œuvre dans le procédé de synthèse ne génèrent aucun sel à séparer et à éliminer. Ces réactions ne génèrent dans le procédé de synthèse que des co-produits qui sont de l'eau et/ou des alcools légers.

En ce sens l'invention, se distingue avantageusement de l'art antérieur.

Ainsi, la présente invention propose un procédé de synthèse d'au moins la N,N'-diméthyl-N,N'-di(2-hydroxyéthyl)-1,6-hexanediamine de formule (I₂), et appartenant à la famille des 1,6-hexanediamines tertiaires partiellement N-hydroxyéthylées de formule générale (I) suivante : 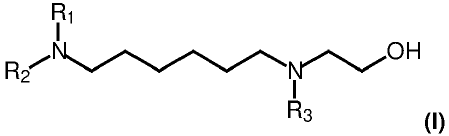 dans laquelle les radicaux R₁, R₂, R₃ sont chacun choisi indifféremment parmi un radical méthyle et un radical hydroxyéthyle, et au moins un radical parmi R₁, R₂, et R₃ est un radical méthyle.
Le procédé comporte deux étapes réactionnelles, et aucune des deux étapes réactionnelles ne comporte de réactif halogéné.
Le procédé comporte au moins une première réaction entre un premier composé précurseur exempt d'atome halogène et un deuxième composé précurseur exempt d'atome d'halogène, le premier composé précurseur comportant un squelette carboné formé par un enchaînement linéaire de 6 atomes de carbone dans lequel les 4 atomes de carbone centraux sont liés chacun à 2 atomes d'hydrogène et les atomes de carbone situés en alpha et en oméga ne sont pas liés à un atome d'halogène.

Selon l'invention, on forme au moins la N,N'-diméthyl-N,N'-di(2-hydroxyéthyl)-1,6-hexanediamine de formule (I₂) suivante :

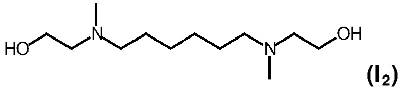

Selon l'invention, le premier composé précurseur est la 1,6-hexanediamine et le deuxième composé précurseur est l'oxyde d'éthylène. Le procédé comprend alors :
- une première étape de réaction entre la 1,6-hexanediamine et l'oxyde d'éthylène pour former au moins un composé intermédiaire choisi dans la liste comprenant la N-(2-hydroxyéthyl)-1,6-hexanediamine, la N-N'-di(2-hydroxyéthyl)-1,6-hexanediamine, la N,N-di(2-hydroxyéthyl)-1,6-hexanediamine, et la N-N-N'-tri(2-hydroxyéthyl)-1,6-hexanediamine ;
- une deuxième étape de méthylation des fonctions amines primaires ou secondaires dudit au moins un composé intermédiaire pour former au moins le composé selon la formule (I₂).

La deuxième étape de méthylation est opérée par réaction entre ledit composé intermédiaire, du formaldéhyde et de l'hydrogène en présence d'un catalyseur d'hydrogénation, ou par réaction entre ledit composé intermédiaire, du formaldéhyde et de l'acide formique selon la réaction de Eichweiller-Clarke.

Avantageusement, on recycle à la première étape de réaction la 1,6-hexanediamine résiduelle n'ayant pas réagi à l'issue de ladite première étape de réaction, après séparation par distillation de ladite 1,6-hexanediamine dudit au moins composé intermédiaire.

Avantageusement, le rapport molaire d'oxyde d'éthylène par rapport à la 1,6-hexanediamine est inférieur ou égal à 3/1, de préférence inférieur ou égal à 2,5/1.

Le procédé peut en outre comporter à l'issue de la première étape de réaction au moins une étape de séparation, de préférence par distillation, d'au moins un composé intermédiaire, pour former un composé spécifique répondant à la formule générale (I) ou une combinaison de composés spécifiques répondant à la formule générale (I) à l'issue de la deuxième étape de méthylation.

D'autres objets et avantages de l'invention apparaîtront à la lecture de la description qui suit d'exemples de réalisations particuliers de l'invention, donnés à titre d'exemples non limitatifs, la description étant faite en référence aux figures annexées décrites ci-après.

### Brève description des figures

La figure 1 représente un procédé de synthèse de composés de formule générale (I) à partir de 1,6-hexanediamine selon un mode de réalisation de l'invention.
La figure 2 représente la synthèse de N,N'-diméthyl-N-N'-di(2-hydroxyéthyl)-1,6-hexanediamine à partir de 1,6-hexanediol.
La figure 3 représente la synthèse de N,N'-diméthyl-N-N'-di(2-hydroxyéthyl)-1,6-hexanediamine à partir d'adipaldéhyde (3 étapes).
La figure 4 représente la synthèse de N,N'-diméthyl-N-N'-di(2-hydroxyéthyl)-1,6-hexanediamine à partir d'adipaldéhyde (une étape).
La figure 5 représente la synthèse de N,N'-diméthyl-N-N'-di(2-hydroxyéthyl)-1,6-hexanediamine à partir d'adipate de diméthyle ou d'acide adipique (2 étapes).
La figure 6 représente la synthèse de N,N'-diméthyl-N-N'-di(2-hydroxyéthyl)-1,6-hexanediamine à partir d'adipate de diméthyle ou d'acide adipique (3 étapes).
La figure 7 représente un autre procédé de synthèse de composés de formule générale (I) à partir de 1,6-hexanediamine.

Dans les figures, l'abréviation "cat" signifie catalyseur, et les flèches représentent des étapes de réaction. Il s'agit de schémas réactionnels. Les illustrations du procédé de synthèse selon l'invention ne comportent pas l'ensemble des composantes nécessaires à sa mise en œuvre. Seuls les éléments nécessaires à la compréhension de l'invention y sont représentés, l'homme du métier étant capable de compléter cette représentation pour mettre en œuvre l'invention.

### Description détaillée de l'invention

La présente invention porte sur la synthèse de N,N'-diméthyl-N-N'-di(2-hydroxyéthyl)-1,6-hexanediamine de formule **(I₂),** appartenant aux 1,6-hexanediamines tertiaires partiellement N-hydroxyéthylées qui répondent à la formule générale **(I)** suivante : 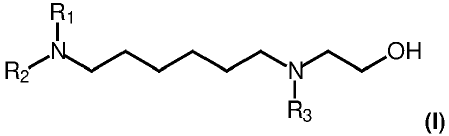 dans laquelle :
les radicaux R₁, R₂, R₃ sont chacun choisi indifféremment parmi un radical méthyle et un radical hydroxyéthyle, et
au moins un radical parmi R₁, R₂, et R₃ est un radical méthyle.

Dans la formule générale (I), les radicaux R₁ et R₃ sont des radicaux méthyle et le radical R₂ est un radical hydroxyéthyle, ou les radicaux R₂ et R₃ sont des radicaux méthyle et le radical R₁ est un radical hydroxyéthyle.

Au moins la 1,6-hexanediamine tertiaire partiellement N-hydroxyéthylée de formule **(I₂)** suivante est synthétisée selon l'invention :
- la N,N,N'-triméthyl-N'-(2-hydroxyéthyl)-1,6-hexanediamine de formule **(I₁)** suivante : 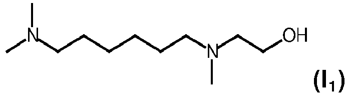
- la N,N'-diméthyl-N,N'-di(2-hydroxyéthyl)-1,6-hexanediamine de formule **(I₂)** suivante : 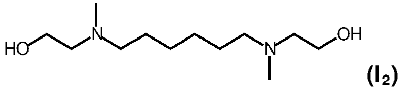
- la N,N-diméthyl-N',N'-di(2-hydroxyéthyl)-1,6-hexanediamine de formule **(I₃)** suivante : 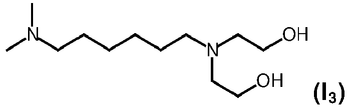
- la N-méthyl-N,N',N'-tri(2-hydroxyéthyl)-1,6-hexanediamine de formule **(I₄)** suivante :

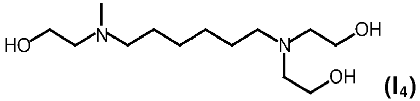

### Synthèse à partir de la 1,6-hexanediamine selon l'invention

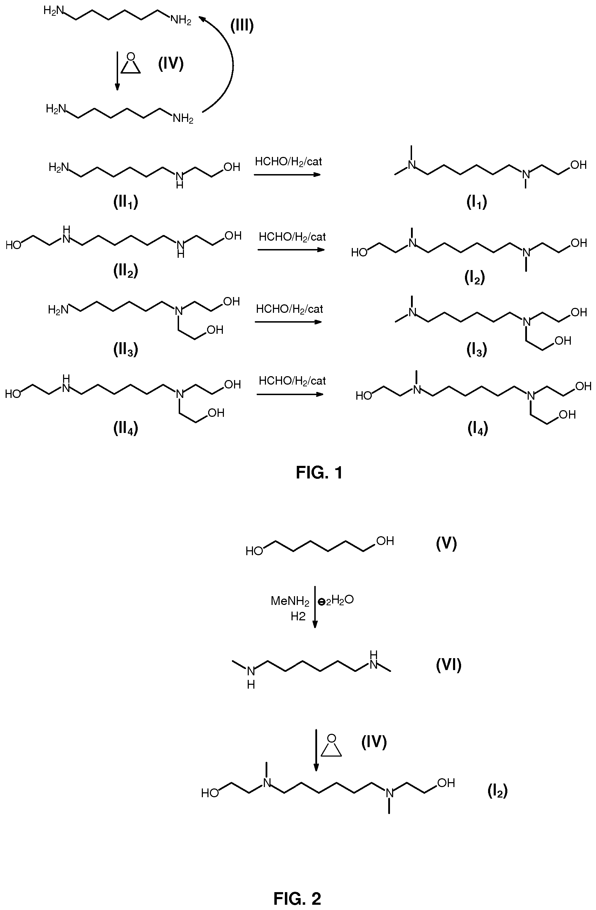

La figure 1 illustre la synthèse d'au moins un composé de formule générale **(I)** à partir de la 1,6-hexanediamine, de formule **(III).**

La synthèse consiste en la succession de deux étapes de réactions à partir de 1,6-hexanediamine.

La première étape consiste à faire réagir la 1,6-hexanediamine avec de l'oxyde d'éthylène (formule **(IV)**) en quantité et dans des conditions adéquates afin d'obtenir soit une 1,6-hexanediamine partiellement éthoxylée telle la N-(2-hydroxyéthyl)-1,6-hexanediamine (formule **II₁**) ou la N-N'-di(2-hydroxyéthyl)-1,6-hexanediamine (formule **II₂**) ou la N,N-di(2-hydroxyéthyl)-1,6-hexanediamine (formule **II₃**) ou la N-N-N'-tri(2-hydroxyéthyl)-1,6-hexanediamine (formule **II₄**), soit un mélange de ces molécules. Ces 1,6-hexanediamines partiellement éthoxylées, de formule (**II₁**) à (**II₄**) sont des produits intermédiaires dans ce mode de synthèse.

Les conditions de réaction sont adaptées afin de ne pas obtenir de N,N,N',N'-tétra(2-hydroxyéthy)-1,6-hexanediamine et de minimiser la quantité de 1,6-hexanediamine résiduelle. Pour cela, il est avantageux d'opérer avec un rapport molaire d'oxyde d'éthylène par rapport à la 1,6-hexanediamine n'excédant pas 3/1, et de préférence n'excédant pas 2,5/1. Le choix du rapport molaire entre l'oxyde d'éthylène et la 1,6-hexanediamine conditionne la composition des produits obtenus.

Lorsque de la 1,6-hexanediamine résiduelle est présente, elle peut être éliminée du milieu par exemple par distillation et éventuellement recyclée.

Lorsque de la N,N,N',N'-tétra(2-hydroxyéthy)-1,6-hexanediamine est obtenue, elle peut être éliminée du milieu par exemple par distillation.

Lorsqu'un mélange de composés intermédiaires est obtenu, il peut être utilisé tel quel pour effectuer la seconde étape ou bien il peut être soumis à une séparation, par exemple par distillation, afin d'obtenir un des composés selon la formule générale **(I)** ou une combinaison de ces composés (formules **I₁** à **I₄**) à l'issue de la deuxième étape.

La deuxième étape consiste en la méthylation des fonctions amines primaires ou secondaires des composés intermédiaires (de formules **II₁** à **II₄**) obtenus lors de la première étape pour conduire à la N,N,N'-triméthyl-N'-(2-hydroxyéthyl)-1,6-hexanediamine (formule **I₁**) et/ou à la N,N'-diméthyl-N-N'-di(2-hydroxyéthyl)-1,6-hexanediamine (formule **I₂**) et/ou à la N,N-diméthyl-N'-N'-di(2-hydroxyéthyl)-1,6-hexanediamine (formule **I₃**) et/ou à la N-méthyl-N-N'-N'-tri(2-hydroxyéthyl)-1,6-hexanediamine (formule **I₄**).

La méthylation des fonctions amines peut s'effectuer par tous les moyens connus de l'homme du métier notamment par réaction de formaldéhyde (HCHO) et d'hydrogène (H₂) en présence d'un catalyseur d'hydrogénation approprié (tel que représenté sur la figure 1) ou par exemple par réaction de formaldéhyde et d'acide formique selon la réaction dite de Eichweiller-Clarke.

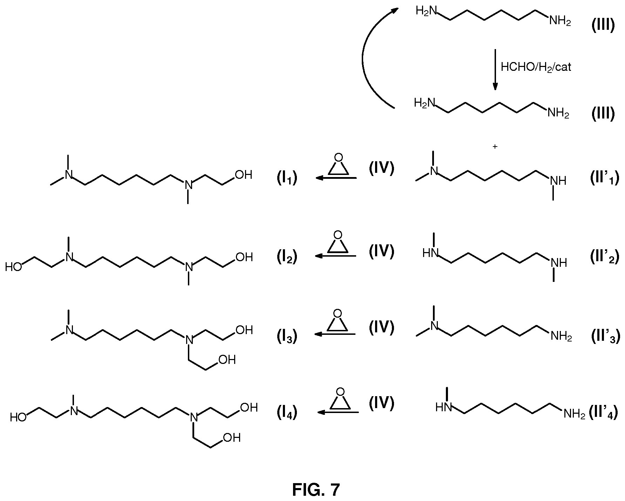

Selon une voie de synthèse à partir de la de 1,6-hexanediamine, les étapes d'éthoxylation et de méthylation peuvent être inversées par rapport à la voie selon l'invention décrite précédemment et illustrée à la figure 1. Dans ce cas, une première étape de méthylation partielle de la 1,6-hexanediamine par réaction avec du formaldéhyde et de l'hydrogène en présence d'un catalyseur d'hydrogénation, conduit à la N-méthyl-1,6-hexanediamine (formule (**II'₄**)) et/ou à la N,N-diméthyl-1,6-hexanediamine (formule (**II'₃**)) et/ou à la N,N'-diméthyl-1,6-hexanediamine (formule (**II'₂**)) et/ou à la N,N,N'-triméthyl-1,6-hexanediamine (formule (**II'₁**)). Puis dans une deuxième étape les fonctions amines primaires et secondaires sont éthoxylée par réaction avec de l'oxyde d'éthylène (formule (**IV**)). Cette voie est illustrée à la figure 7.

### Synthèse à partir du 1,6-hexanediol

Un composé de formule générale **(I)** peut être synthétisé à partir du 1,6-hexanediol (formule **(V)).**

La figure 2 illustre la synthèse d'un exemple de composé selon la formule générale **(I)**, la N,N'-diméthyl-N,N'-di(2-hydroxyéthyl)-1,6-hexanediamine de formule **(I₂)**, à partir de la 1,6-hexanediol de formule **(V).**

La synthèse consiste en la succession de deux étapes de réactions : la condensation du 1,6-hexanediol avec de la méthylamine (MeNH₂) utilisée généralement en excès en présence d'hydrogène et d'un catalyseur approprié pour conduire au N,N'-diméthyl-1,6-hexanediamine (formule **(VI)**) qui est ensuite transformé en N,N'-diméthyl-N-N'-di(2-hydroxyéthyl)-1,6-hexanediamine (formule **(I₂)**) par une réaction d'éthoxylation avec de l'oxyde d'éthylène (formule **(IV)**).

### Synthèse à partir de l'adipaldéhyde

Un composé de formule générale (I) peut être synthétisé à partir d'adipaldéhyde. *Synthèse en 3 étapes de réactions*

Un composé de formule générale **(I)** peut être synthétisé en trois étapes de réactions à partir de l'adipaldéhyde (formule **(VII)**).

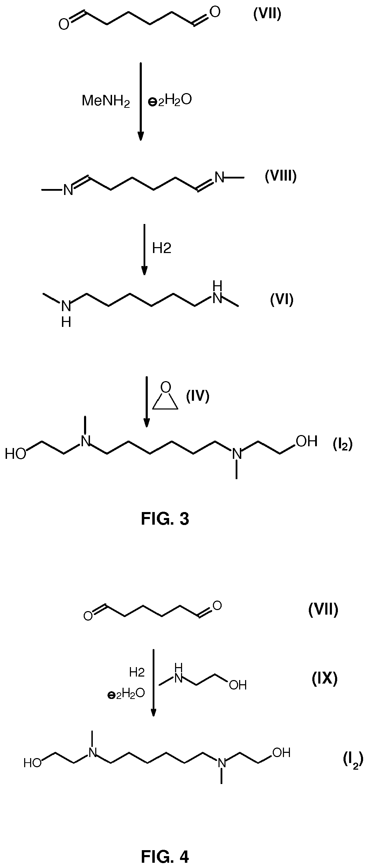

La figure 3 illustre la synthèse d'un exemple de composé selon la formule générale **(I)**, la N,N'-diméthyl-N,N'-di(2-hydroxyéthyl)-1,6-hexanediamine de formule **(I₂)**, à partir de l'adipaldéhyde de formule **(VII)**, selon un procédé de synthèse en trois étapes réactionnelles.

La synthèse consiste en la succession de trois étapes de réactions.

La première étape consiste en une réaction condensation de l'adipaldéhyde (formule **(VII)**) avec de la méthylamine (MeNH₂) pour conduire au 1,6-bis(methylimino)-hexane (formule **(VIII)**).

Une deuxième étape de réaction consiste en la réduction du 1,6-bis(methylimino)-hexane (formule **(VIII)**) en N,N'-diméthyl-1,6-hexanediamine (formule **(VI)**).

Une troisième étape de réaction consiste en l'éthoxylation de la N,N'-diméthyl-1,6-hexanediamine (formule **(VI)**) avec de l'oxyde d'éthylène (formule **(IV)**) pour former de la N,N'-diméthyl-N-N'-di(2-hydroxyéthyl)-1,6-hexanediamine (formule **(I₂)**).

### Synthèse en 1 étape de réaction

Un composé de formule générale (I) peut être synthétisé en une seule étape de réaction à partir de l'adipaldéhyde (formule **(VII)**).

La figure 4 illustre la synthèse d'un exemple de composé selon la formule générale (I), la N,N'-diméthyl-N,N'-di(2-hydroxyéthyl)-1,6-hexanediamine de formule **(I₂)**, à partir de l'adipaldéhyde (formule **(VII)**), selon un procédé de synthèse en une étape réactionnelle.

La synthèse consiste en la condensation de l'adipaldéhyde (formule **(VII)**) avec le N-méthyl-2-aminoéthanol (formule **(IX)**) en présence d'hydrogène et d'un catalyseur d'hydrogénation adéquat pour conduire à la N,N'-diméthyl-N-N'-di(2-hydroxyéthyl)-1,6-hexanediamine (formule **(I₂)**).

Cette transformation, aussi appelée amination réductrice, résulte de la succession de deux réactions qui sont la condensation d'une amine secondaire avec un aldéhyde conduisant à une enamine, puis l'hydrogénation en amine tertiaire de cette dernière.

### Synthèse à partir de l'acide adipique ou d'un diester de l'acide adipique

Un composé de formule générale (I) peut être synthétisé à partir de l'acide adipique ou d'un diester de l'acide adipique.

### Synthèse en 2 étapes de réactions

Un composé de formule générale **(I)** peut être synthétisé en deux étapes de réactions à partir de l'acide adipique (formule **(XI)**) ou d'un diester de l'acide adipique tel que l'adipate de diméthyle (formule **(X)).**

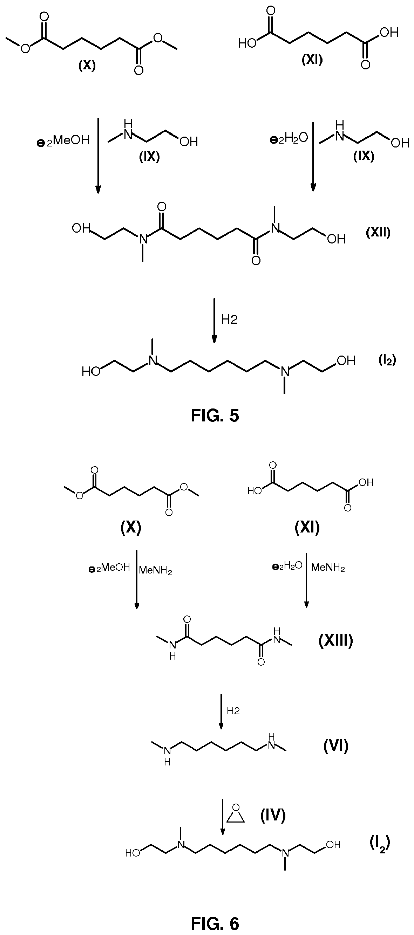

La figure 5 illustre la synthèse d'un exemple de composé selon la formule générale **(I)**, la N,N'-diméthyl-N,N'-di(2-hydroxyéthyl)-1,6-hexanediamine de formule **(I₂)**, à partir de de l'acide adipique (formule **(XI)**) ou d'un diester de l'acide adipique, tel que l'adipate de diméthyle (formule **(X)),** selon un procédé de synthèse en deux étapes réactionnelles.

Une première étape consiste en la réaction de condensation de N-méthyl-2-aminoéthanol (formule **(IX)**) avec soit de l'acide adipique (formule **(XI)**) soit un diester de l'acide adipique tel par exemple sans être limitatif l'adipate de diméthyle (formule **(X))** ou l'adipate de diéthyle. Le produit de condensation obtenu est un diamide le N,N'-diméthyl-N,N'-di(2-hydroxyéthyl)-adipamide (formule **(XII)**).

La deuxième étape consiste en la réduction des fonctions amides du N,N'-diméthyl-N,N'-di(2-hydroxyéthyl)-adipamide (formule **(XII)**) en fonctions amines tertiaires pour conduire à la N,N'-diméthyl-N-N'-di(2-hydroxyéthyl)-1,6-hexanediamine (formule **(I₂)**).

Cette réduction peut s'effectuer par tous les moyens connus telle la réduction par hydrogénation catalytique ou l'action d'hydrures tels l'hydrure de lithium et d'aluminium.

### Synthèse en 3 étapes de réactions

Un composé de formule générale **(I)** peut être synthétisé en trois étapes de réactions à partir de de l'acide adipique (formule **(XI)**) ou d'un diester de l'acide adipique tel que l'adipate de diméthyle (formule **(X)**).

La figure 6 illustre la synthèse d'un exemple de composé selon la formule générale **(I)**, la N,N'-diméthyl-N,N'-di(2-hydroxyéthyl)-1,6-hexanediamine de formule **(I₂)**, à partir de l'acide adipique (formule **(XI)**) ou d'un diester de l'acide adipique, tel que l'adipate de diméthyle (formule **(X)**), selon un procédé de synthèse en trois étapes réactionnelles.

Une première étape consiste en la condensation de la méthylamine (MeNH₂) avec soit de l'acide adipique (formule **(XI)**) soit un diester de l'acide adipique tel par exemple sans être limitatif l'adipate de diméthyle (formule **(X))** ou l'adipate de diéthyle. Le produit de condensation obtenu est un diamide : le N,N'-diméthyl-N,N'-adipamide de formule **(XIII)**.

Une deuxième étape consiste en la réduction des fonctions amides du N,N'-diméthyl-N,N'-adipamide de formule **(XIII)** en fonctions amines tertiaires pour conduire à la N,N'-diméthyl-1,6-hexanediamine (formule **(VI)**). Cette réduction peut s'effectuer par tous les moyens connus telle la réduction par hydrogénation catalytique ou l'action d'hydrures tels l'hydrure de lithium et d'aluminium.

Une troisième étape consiste à éthoxyler les fonctions amines secondaires de la N,N'-diméthyl-1,6-hexanediamine (formule **(VI)**) avec de l'oxyde d'éthylène (formule **(IV)**) pour conduire à la N,N'-diméthyl-N-N'-di(2-hydroxyéthyl)-1,6-hexanediamine (formule **(I₂)**).

### Annexe : liste des noms de composés et leur formule apparaissant dans les figures :

| **Réf.** | **Nom** | **Formule** |
|---|---|---|
| **(I₁)** | N,N,N'-triméthyl-N'-(2-hydroxyéthyl)-1,6-hexanediamine de formule **(I₁)** suivante | 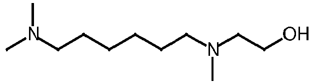 |
| **(I₂)** | N,N'-diméthyl-N,N'-di(2-hydroxyéthyl)-1,6-hexanediamine | 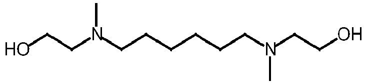 |
| **(I₃)** | N,N-diméthyl-N',N'-di(2-hydroxyéthyl)-1,6-hexanediamine | 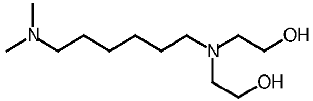 |
| **(I₄)** | la N-méthyl-N,N',N'-tri(2-hydroxyéthyl)-1,6-hexanediamine | 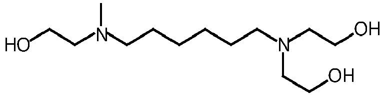 |
| **(II₁)** | N-(2-hydroxyéthyl)-1,6-hexanediamine | 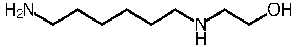 |
| **(II₂)** | N-N'-di(2-hydroxyéthyl)-1,6-hexanediamine | 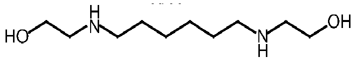 |
| **(II₃)** | N,N-di(2-hydroxyéthyl)-1,6-hexanediamine | 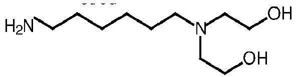 |
| **(II₄)** | N-N-N'-tri(2-hydroxyéthyl)-1,6-hexanediamine | 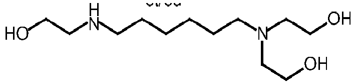 |
| **(III)** | 1,6-Hexanediamine | 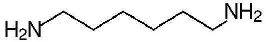 |
| **(IV)** | Oxyde d'éthylène | 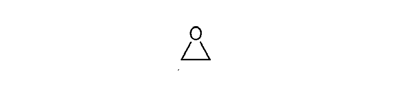 |
| **(V)** | 1,6-Hexanediol | 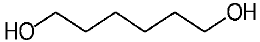 |
| **(VI)** | N,N'-diméthyl-1,6-hexanediamine | 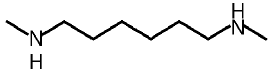 |
| **(VII)** | adipaldéhyde | 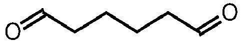 |
| **(VIII)** | 1,6-bis(methylimino)-hexane | 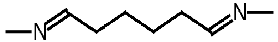 |
| **(IX)** | N-méthyl-2-aminoéthanol | 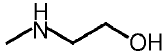 |
| **(X)** | adipate de diméthyle | 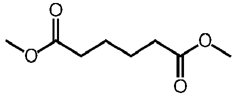 |
| **(XI)** | acide adipique | 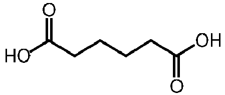 |
| **(XII)** | N,N'-diméthyl-N,N'-di(2-hydroxyéthyl)-adipamide | 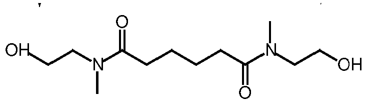 |
| **(XIII)** | N,N'-diméthyl-N,N'-adipamide | 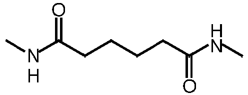 |
| **(II'₁)** | N,N,N'-triméthyl-1,6-hexanediamine | 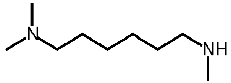 |
| **(II'₂)** | N,N'-diméthyl-1,6-hexanediamine | 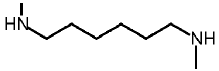 |
| **(II'₃)** | N,N-diméthyl-1,6-hexanediamine | 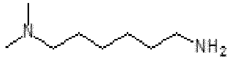 |
| **(II'₄)** | N-méthyl-1,6-hexanediamine | 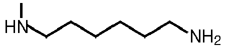 |

## Revendications

1. Procédé de synthèse d'au moins la N,N'-diméthyl-N,N'-di(2-hydroxyéthyl)-1,6-hexanediamine de formule (I₂) suivante : 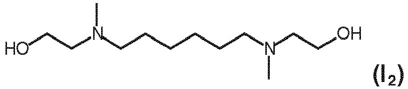
comportant deux étapes réactionnelles, et
comportant au moins une première réaction entre un premier composé précurseur exempt d'atome halogène et un deuxième composé précurseur exempt d'atome d'halogène,
ledit premier composé précurseur comportant un squelette carboné formé par un enchaînement linéaire de 6 atomes de carbone dans lequel les 4 atomes de carbone centraux sont liés chacun à 2 atomes d'hydrogène et les atomes de carbone situés en alpha et en oméga ne sont pas liés à un atome d'halogène,
et dans lequel aucune des deux étapes réactionnelles ne comporte de réactif organique halogéné,
le premier composé précurseur étant la 1,6-hexanediamine et le deuxième composé précurseur étant l'oxyde d'éthylène, ledit procédé comprenant :
- une première étape de réaction entre la 1,6-hexanediamine et l'oxyde d'éthylène pour former au moins un composé intermédiaire choisi dans la liste comprenant la N-(2-hydroxyéthyl)-1,6-hexanediamine, la N-N'-di(2-hydroxyéthyl)-1,6-hexanediamine, la N,N-di(2-hydroxyéthyl)-1,6-hexanediamine, et la N-N-N'-tri(2-hydroxyéthyl)-1,6-hexanediamine ;
- une deuxième étape de méthylation des fonctions amines primaires ou secondaires dudit au moins un composé intermédiaire pour former au moins la N,N'-diméthyl-N,N'-di(2-hydroxyéthyl)-1,6-hexanediamine,
ladite deuxième étape de méthylation étant opérée par réaction entre ledit composé intermédiaire, du formaldéhyde et de l'hydrogène en présence d'un catalyseur d'hydrogénation, ou par réaction entre ledit composé intermédiaire, du formaldéhyde et de l'acide formique selon la réaction de Eichweiller-Clarke.

2. Procédé selon la revendication 1, dans lequel on recycle à la première étape de réaction la 1,6-hexanediamine résiduelle n'ayant pas réagi à l'issue de ladite première étape de réaction, après séparation par distillation de ladite 1,6-hexanediamine dudit au moins composé intermédiaire.

3. Procédé selon l'une des revendications 1 ou 2, dans lequel le rapport molaire d'oxyde d'éthylène par rapport à la 1,6-hexanediamine est inférieur ou égal à 3/1, de préférence inférieur ou égal à 2,5/1.

4. Procédé selon l'une des revendications 1 à 3, comportant en outre à l'issue de la première étape de réaction au moins une étape de séparation, de préférence par distillation, d'au moins un composé intermédiaire, pour former un composé spécifique répondant à la formule générale (I) ou une combinaison de composés spécifiques répondant à la formule générale (I) à l'issue de la deuxième étape de méthylation.

## Patentansprüche

1. Verfahren zur Synthese von mindestens N,N'-Dimethyl-N,N'-di(2-hydroxyethyl)-1,6-hexandiamin der folgenden Formel (I₂): 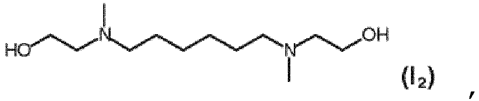
umfassend zwei Reaktionsschritte und
umfassend mindestens eine erste Reaktion zwischen einer ersten Vorläuferverbindung ohne Halogenatom und einer zweiten Vorläuferverbindung ohne Halogenatom,
wobei die erste Vorläuferverbindung ein Kohlenstoffskelett umfasst, das von einer geraden Aneinanderreihung von 6 Kohlenstoffatomen gebildet wird, worin 4 zentrale Kohlenstoffatome jeweils an 2 Wasserstoffatome gebunden sind und die alpha- und omega-ständigen Kohlenstoffatome nicht an ein Halogenatom gebunden sind,
und wobei keiner der beiden Reaktionsschritte einen halogenierten organischen Reaktanten einschließt,
wobei es sich bei der ersten Vorläuferverbindung um 1,6-Hexandiamin und bei der zweiten Vorläuferverbindung um Ethylenoxid handelt, wobei das Verfahren Folgendes umfasst:
- einen ersten Reaktionsschritt zwischen 1,6-Hexandiamin und Ethylenoxid, um mindestens eine Zwischenverbindung herzustellen, ausgewählt aus der N-(2-Hydroxyethyl)-1,6-hexandiamin, N-N'-Di(2-hydroxyethyl)-1,6-hexandiamin, N,N-Di(2-hydroxyethyl)-1,6-hexandiamin und N-N-N'-Tri(2-hydroxyethyl)-1,6-hexandiamin umfassenden Liste;
- einen zweiten Schritt der Methylierung der primären oder sekundären Aminfunktionen der mindestens einen Zwischenverbindung, um mindestens N,N'-Dimethyl-N,N'-di(2-hydroxyethyl)-1,6-hexandiamin herzustellen,
wobei der zweite Schritt der Methylierung durch Reaktion zwischen der Zwischenverbindung, Formaldehyd und Wasserstoff in Gegenwart eines Hydrierungskatalysators oder durch Reaktion zwischen der Zwischenverbindung, Formaldehyd und Ameisensäure nach der Eschweiler-Clarke-Reaktion durchgeführt wird.

2. Verfahren nach Anspruch 1, wobei man das restliche 1,6-Hexandiamin, das am Ende des ersten Reaktionsschritts nicht umgesetzt ist, nach Abtrennung des 1,6-Hexandiamins mittels Destillation von mindestens der Zwischenverbindung in den ersten Reaktionsschritt rezykliert.

3. Verfahren nach einem der Ansprüche 1 oder 2, wobei das Molverhältnis von Ethylenoxid zu 1,6-Hexandiamin kleiner als oder gleich 3/1, vorzugsweise kleiner als oder gleich 2,5/1, ist.

4. Verfahren nach einem der Ansprüche 1 bis 3, das außerdem am Ende des ersten Reaktionsschritts mindestens einen Schritt der Abtrennung, vorzugsweise mittels Destillation, mindestens einer Zwischenverbindung umfasst, um am Ende des zweiten Schritts der Methylierung eine spezifische, der allgemeinen Formel (I) entsprechende Verbindung oder eine Kombination von spezifischen, der allgemeinen Formel (I) entsprechenden Verbindungen herzustellen.

## Claims

1. Method for synthesizing at least N,N'-dimethyl-N,N'-di(2-hydroxyethyl)-1,6-hexanediamine of formula (I₂) below: 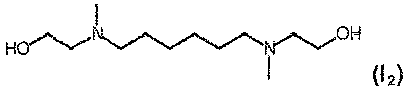
comprising two reaction steps, and
comprising at least a first reaction between a first halogen atom-free precursor compound and a second halogen atom-free precursor compound,
said first precursor compound comprising a carbon-based backbone formed by a linear chain of 6 carbon atoms in which the central 4 carbon atoms are each bonded to 2 hydrogen atoms and the carbon atoms located in the alpha and omega positions are not bonded to a halogen atom, and in which neither of the two reaction steps comprises a halogenated organic reactant,
the first precursor compound being 1,6-hexanediamine and the second precursor compound being ethylene oxide, said method comprising:
- a first step of reaction between 1,6-hexanediamine and ethylene oxide to form at least one intermediate compound selected from the group comprising N-(2-hydroxyethyl)-1,6-hexanediamine, N,N'-di(2-hydroxyethyl)-1,6-hexanediamine, N,N-di(2-hydroxyethyl)-1,6-hexanediamine and N,N,N'-tri(2-hydroxyethyl)-1,6-hexanediamine;
- a second step of methylation of the primary or secondary amine functions of said at least one intermediate compound to form at least N,N'-dimethyl-N,N'-di(2-hydroxyethyl)-1,6-hexanediamine,
said second methylation step being carried out by reaction between said intermediate compound, formaldehyde and hydrogen in the presence of a hydrogenation catalyst, or by reaction between said intermediate compound, formaldehyde and formic acid according to the Eschweiler-Clarke reaction.

2. Method according to Claim 1, in which the residual 1,6-hexanediamine that has not reacted at the end of the first reaction step is recycled to said first reaction step, after separation by distillation of said 1,6-hexanediamine from said at least one intermediate compound.

3. Method according to either of Claims 1 and 2, in which the molar ratio of ethylene oxide with respect to to 1,6-hexanediamine is less than or equal to 3/1, preferably less than or equal to 2.5/1.

4. Method according to one of Claims 1 to 3, further comprising, at the end of the first reaction step, at least one step of separation, preferably by distillation, of at least one intermediate compound, to form a specific compound corresponding to the general formula (I) or a combination of specific compounds corresponding to the general formula (I) at the end of the second methylation step.
